# EUROPEAN PATENT APPLICATION

(11) **EP 4 184 149 A1**
(43) Date of publication of application: **24.05.2023**
(21) Application number: 21214397.8
(22) Date of filing: 14.12.2021
(51) Int. Cl.: G01N 21/64, G01N 21/05, B01F 23/00

(54) **METHOD FOR PROVIDING QUANTITATIVE INFORMATION OF TARGETS AND DEVICE USING THE SAME**

(30) Priority: 18.11.2021 KR 20210159529
(71) Applicant: Korea Advanced Institute of Science and Technology, Daejeon 34141 (KR)
(72) Inventor: LEE, Kyoung Gyun, 34140 Daejeon (KR); LEE, Seok Jae, 34085 Daejeon (KR); BAE, Nam Ho, 34067 Daejeon (KR); RHO, Dong Gee, 34184 Daejeon (KR); LEE, Tae Jae, 28636 Chungcheongbuk-do (KR); LEE, Moon Keun, 34085 Daejeon (KR); PARK, Yoo Min, 28330 Chungcheongbuk-do (KR)
(74) Representative: Fabry, Bernd

(57) **Abstract**

A method for providing quantitative information for targets and a device using the same according to an exemplary embodiment of the present disclosure are provided. A quantitative information providing method for targets according to the exemplary embodiment of the present disclosure includes flowing a plurality of microdroplets into a chamber or a channel including a detection region such that the plurality of microdroplets including targets is present as a single layer, acquiring a single layer of microdroplets in which the plurality of microdroplets is present as a single layer, and providing quantitative data of targets based on the single layer image of the microdroplets, and the detection region has a height which is one time to about two times of a diameter of the plurality of microdroplets and is defined as a region in which the plurality of microdroplets is dispersed in a plurality of columns to fill the detection region.

## Description

### CROSS-REFERENCE TO RELATED APPLICATIONS

This application claims the priority of Korean Patent Application No. 10-2021-0159529 filed on November 18, 2021, in the Korean Intellectual Property Office, the disclosure of which is incorporated herein by reference.

### BACKGROUND

### Field

The present disclosure relates to a method for providing quantitative information of targets and a device using the same.

### Description of the Related Art

A digital assay refers to a method that segments a target into thousands of micro structures, checks a signal according to the presence/absence of the targets, and then quantifies the signal using a Poisson process. Such a digital assay has advantages of having a higher accuracy and sensitivity than the other analysis methods and enabling absolute quantification without the need for standard samples.

Examples of the digital assay method include digital polymerase chain reaction (digital PCR), a digital enzyme-linked immunosorbent assay (digital ELISA), a digital proximity ligation assay (digital PLA), and a digital loop-mediated isothermal amplification assay (digital LAMP), and these digital assay methods have been applied to various diagnosis.

For example, the digital PCR is widely utilized for the purpose of research development or diagnosis in the fields of life science, genetic engineering, and medicine.

Specifically, the PCR is a molecular biology technology for replicating DNA from an isolated biological sample and may be usually used for various tasks such as diagnosis of infectious diseases, detection of hereditary diseases, identification of genetic fingerprints, gene cloning, paternity testing, genotyping identification, gene sequencing test, and DNA computing.

Specifically, the digital polymerase chain reaction (dPCR) technique is a genetic testing method which segments an existing sample into microdroplets having a volume of nanoliters and observes the fluorescence variation in a target therein. Such digital PCR has a very high sensitivity and enables absolute quantitative analysis so that it has a high applicability to gene analysis, biomarker development, and gene sequencing.

In the meantime, in some digital assay techniques including digital PCR, a configuration and a procedure for spacing between microdroplets which pass through a reader are essential to avoid issues such as optical coherence in the photo detection. That is, according to this method, an optical peak for each microdroplet which passes through the reader is measured and quantitative information of the targets is determined therefrom so that it takes a long time to analyze.

Accordingly, the development of a system for providing quantitative information for a new target which is capable of overcoming the limitations of the digital assay of the related art and analyzing targets in the sample with a high precision is consistently demanded.

The background of the present disclosure is described for easier understanding of the present disclosure. It should not be understood to admit the matters described in the background of the present disclosure as a prior art.

Related Art Document: US 20140221239 A1

### SUMMARY

In the meantime, the inventors of the present disclosure have noted a limitation of the digital assay technique of the related art in that the quantitative analysis of targets is performed while flowing microdroplets on which a specific step such as PCR is completed to a channel type detecting unit, based on a geometrical optical reader.

To be more specific, the inventors of the present disclosure have recognized that according to the digital assay technique of the related art, as an intensity of fluorescent material for each microdroplet flowing to a detecting unit is analyzed, it takes a long time to analyze, an expensive photo multiplier tube (PMT) is necessary, reproduction of the liquid droplets may be requested, and an error in the quantitative analysis may occur due to the optical coherence.

The inventors of the present disclosure have noted that image based quantitative analysis on microdroplets may solve the above-mentioned problems.

Specifically, the inventors of the present disclosure have noted that an image for microdroplets including targets is acquired to perform quantitative analysis so that the limitation of the digital assay of the related art which detects a peak in a specific wavelength range to perform the quantitative analysis may be overcome.

As a result, the inventors of the present disclosure have come to develop a new quantitative information providing system based on a microdroplet image.

To be more specific, the inventors of the present disclosure may design the quantitative information providing system to flow the microdroplets to a chamber or a channel structurally designed such that the microdroplets are present as a single layer, and then perform specific reaction or analysis (for example, PCR, ELISA, PLA, LAMP, or the like) and acquire a single layer image to detect positive microdroplets including targets.

Moreover, the inventors of the present disclosure may design the quantitative information providing system to flow the microdroplets on which a specific reaction or analysis is completed, to a chamber or a channel designed such that the microdroplets are present as a single layer and then acquires a single layer image to detect positive microdroplets including targets.

That is, the inventors of the present disclosure may recognize that the new quantitative information providing system may quantitatively analyze targets without spacing the microdroplets including the target on which the reaction or analysis is completed.

Specifically, the inventors of the present disclosure have tried to apply a model capable of segmenting positive microdroplets and negative microdroplets in an image using deep learning which segments an image for microdroplets.

As a result, the inventors of the present disclosure may design a new quantitative information providing system which is capable of distinguishing and counting not only positive microdroplets, but also negative microdroplets which do not include target genes in the image for the microdroplets (for example, a fluorescence image) by applying a prediction model.

The inventors of the present disclosure were expected that the microdroplets, moreover, targets in the microdroplets may be precisely quantitatively analyzed using the new quantitative information providing system.

Specifically, the inventors of the present disclosure were expected that the analysis in the unit of microdroplets was allowed to detect a very small amount of viruses and easily distinguish patients with asymptomatic viral infections.

Therefore, an object to be achieved by the present disclosure is to provide a quantitative information providing method for targets and a device for providing quantitative information for targets configured to flow a plurality of microdroplets to a chamber or a channel, receive a single layer image for microdroplets, and quantitatively analyze targets using the same.

Objects of the present disclosure are not limited to the above-mentioned objects, and other objects, which are not mentioned above, can be clearly understood by those skilled in the art from the following descriptions.

In order to achieve the above-described objects, according to an aspect of the present disclosure, a quantitative information providing method for targets is provided.

The method includes flowing a plurality of microdroplets into a chamber or a channel including a detection region such that the plurality of microdroplets including targets is present as a single layer; acquiring a single layer image of microdroplets in which the plurality of microdroplets is present as a single layer; and providing quantitative data of targets based on the single layer image of the microdroplets. At this time, the detection region has a height which is one time to about two times of a diameter of the plurality of microdroplets and is defined as a region in which the plurality of microdroplets is dispersed in a plurality of columns to fill the detection region.

According to a feature of the present disclosure, the chamber or the channel further includes a valve which controls the movement of the plurality of microdroplets and the acquiring of a single layer image of microdroplets further includes: adjusting the valve to stop the plurality of microdroplets in the chamber or the channel; and acquiring a chamber or channel image in which the plurality of stopped microdroplets is present.

According to another feature of the present disclosure, the chamber or the channel further includes an inlet which the plurality of microdroplets flows into the chamber or the channel, and an outlet which the plurality of microdroplets is discharged to the outside of the chamber or the channel. At this time, the flowing includes flowing the plurality of microdroplets from the inlet to the outlet, the acquiring of a single layer image of microdroplets further includes: acquiring a chamber or channel image in which the plurality of microdroplets moves from the inlet to the outlet.

According to still another feature of the present disclosure, the chamber has a tapered structure having a width which is reduced toward the inlet or the outlet.

According to still another feature of the present disclosure, the method further includes, before the flowing, inducing the contact of a sample including the targets and the fluorescent material and oil having an immiscible property with the sample to produce the plurality of microdroplets. Further, the method may further include, after the flowing, controlling a temperature to perform polymerase chain reaction (PCR) of the targets in the plurality of microdroplets in the chamber or the channel. Moreover, the acquiring of a single layer image of microdroplets further includes: acquiring a single layer image of the plurality of microdroplets including the amplified targets.

According to still another feature of the present disclosure, the method further includes, before the flowing, inducing the contact of a sample including the targets and the fluorescent material and oil having an immiscible property with the sample to produce the plurality of microdroplets, and controlling a temperature to perform PCR of the targets in the plurality of microdroplets. At this time, the flowing includes flowing the plurality of microdroplets including the amplified targets to the chamber or the channel without spacing the microdroplets.

According to still another feature of the present disclosure, the providing of quantitative data of the targets further includes: predicting a region of a positive microdroplet and a region of a negative microdroplet based on the single layer image of the microdroplets using an artificial neural network based prediction model configured to segment the regions of the positive microdroplets and the negative microdroplets with the single layer image of the microdroplets as an input; determining the number for the plurality of microdroplets based on the region of the positive microdroplets and the region of the negative microdroplets; and providing quantitative data of targets based on the number of the plurality of microdroplets.

According to still another feature of the present disclosure, the positive microdroplets are defined as microdroplets including the targets and fluorescent materials, the negative microdroplets are defined as microdroplets only including the fluorescent materials or void droplets. At this time, the providing of quantitative data of the targets further includes: determining a concentration of the targets based on the number of positive microdroplets and negative microdroplets.

According to still another feature of the present disclosure, the prediction model is further configured to quantitatively analyze the targets based on the region of the positive microdroplets and the region of the negative microdroplets, and the providing of quantitative data of the targets further includes: determining quantitative data of targets based on the number of the plurality of microdroplets using the prediction model.

In order to achieve the above-described object, a device for providing quantitative information for targets according to the exemplary embodiment of the present disclosure is provided.

The device includes a chamber or a channel which receives a plurality of microdroplets and includes a detection region in which the plurality of microdroplets is present as a single layer; a light source which irradiates light in the detection region of the chamber or the channel; an image sensor configured to provide a single layer image of the microdroplets in which the plurality of microdroplets is present as the single layer in the detection region; and a processor which is operably connected to the image sensor, the processor is configured to provide quantitative data of targets based on the single layer image of the microdroplets. In the meantime, the detection region has a height which is one time to about two times of a diameter of the plurality of microdroplets and is defined as a region in which the plurality of microdroplets is dispersed in a plurality of columns to fill the detection region.

According to a feature of the present disclosure, the chamber or the channel further includes a valve which controls the movement of the plurality of microdroplets. At this time, the processor is configured to adjust the valve to stop the plurality of microdroplets in the chamber or the channel and the image sensor is further configured to acquire a chamber or channel image in which the plurality of stopped microdroplets is present.

According to another feature of the present disclosure, the chamber or the channel further includes an inlet which the plurality of microdroplets flows into the chamber or the channel, and an outlet which the plurality of microdroplets is discharged to the outside of the chamber or the channel. At this time, the processor is configured to flow the plurality of microdroplets from the inlet to the outlet and the image sensor is further configured to acquire a chamber or channel image in which the plurality of microdroplets moves from the inlet to the outlet.

According to still another feature of the present disclosure, the chamber has a tapered structure having a width which is reduced toward the inlet or the outlet.

According to still another feature of the present disclosure, the device further includes a microdroplet producing unit configured to induce the contact of a sample including the targets and the fluorescent material and oil having an immiscible property with the sample to produce the plurality of microdroplets; and a temperature adjusting unit configured to control a temperature to perform polymerase chain reaction (PCR) of the targets in the plurality of microdroplets in the chamber or the channel. At this time, the image sensor is further configured to acquire a single layer image of the microdroplets including an amplified target.

According to still another feature of the present disclosure, the device further includes a microdroplet producing unit configured to induce the contact of a sample including the targets and the fluorescent material and oil having an immiscible property with the sample to produce the plurality of microdroplets; and a temperature adjusting unit configured to control a temperature to perform PCR of the targets in the plurality of microdroplets. At this time, in the chamber or the channel, the plurality of microdroplets including the amplified targets moves or is received without spacing the microdroplets.

According to still another feature of the present disclosure, the processor is further configured to predict a region of a positive microdroplet and a region of a negative microdroplet based on the single layer image of the microdroplets using an artificial neural network based prediction model configured to segment the regions of the positive microdroplets and the negative microdroplets with the single layer image of the microdroplets as an input; determine the number for the plurality of microdroplets based on the region of the positive microdroplets and the region of the negative microdroplets; and provide quantitative data of the targets based on the number for the plurality of microdroplets.

According to still another feature of the present disclosure, the positive microdroplets are defined as microdroplets including the targets and fluorescent materials, the negative microdroplets are defined as microdroplets only including the fluorescent materials or void droplets. At this time, the processor is further configured to determine a concentration of the targets based on the number of positive microdroplets and negative microdroplets.

According to still another feature of the present disclosure, the prediction model is further configured to quantitatively analyze the targets based on the region of the positive microdroplets and the region of the negative microdroplets. At this time, the processor is further configured to determine quantitative data of targets based on the number of the plurality of microdroplets using the prediction model.

Other detailed matters of the exemplary embodiments are included in the detailed description and the drawings.

The present disclosure may provide a new target quantitative system based on a microdroplet image which is capable of detecting a target in the microdroplets without including a unit for spacing between microdroplets.

To be more specific, according to the present disclosure, positive microdroplets including targets may be detected without using a partition for fixing produced microdroplets, such as an array and genes may be quantitatively analyzed with an improved accuracy.

That is, according to the present disclosure, targets may be detected more easily than a serial counting based reading method in which the spacing is essentially required as an optical signal for each of microdroplets which move in the channel is detected.

Moreover, according to the present disclosure, not only positive microdroplets, but also negative microdroplets which do not include targets in an image for microdroplets (for example, a fluorescence image) may be separately provided.

Therefore, the microdroplets including targets may be detected with a high accuracy.

Moreover, the present disclosure provides an absolute quantitative value of targets based on the microdroplet image to perform automatically quantitative analysis without having user's intervention, which may contribute to high advancement of diagnostic techniques.

Specifically, according to the present disclosure, the positive signals are clearly identified to detect a very small amount of virus and easily distinguish patients with asymptomatic viral infections.

The effects according to the present disclosure are not limited to the contents exemplified above, and more various effects are included in the present specification.

### BRIEF DESCRIPTION OF THE DRAWINGS

The above and other aspects, features and other advantages of the present disclosure will be more clearly understood from the following detailed description taken in conjunction with the accompanying drawings, in which:
FIGS. 1A and 1B exemplarily illustrate a structure and configurations of a device for providing quantitative information for targets used for various exemplary embodiments of the present disclosure;
FIGS. 2A to 2C exemplarily illustrate a structure and configurations of a chamber of a device for providing quantitative information for targets used for various exemplary embodiments of the present disclosure; and
FIGS. 3, 4A, 4B, and 5 exemplarily illustrate procedures of providing quantitative information for targets using a device for providing quantitative information for targets according to various exemplary embodiments of the present disclosure.

### DETAILED DESCRIPTION OF THE EMBODIMENT

Advantages of the present disclosure and a method of achieving the advantages and characteristics will be clear by referring to exemplary embodiments described below in detail together with the accompanying drawings. However, the present disclosure is not limited to exemplary embodiment disclosed herein but will be implemented in various forms. The exemplary embodiments are provided by way of example only so that a person of ordinary skilled in the art can fully understand the disclosures of the present disclosure and the scope of the present disclosure. Therefore, the present disclosure will be defined only by the scope of the appended claims.

The shapes, sizes, ratios, angles, numbers, and the like illustrated in the accompanying drawings for describing the exemplary embodiments of the present disclosure are merely examples, and the present disclosure is not limited thereto. Further, in the following description, a detailed explanation of known related technologies may be omitted to avoid unnecessarily obscuring the subject matter of the present disclosure. The terms such as "including," "having," and "consist of" used herein are generally intended to allow other components to be added unless the terms are used with the term "only". Any references to singular may include plural unless expressly stated otherwise.

Components are interpreted to include an ordinary error range even if not expressly stated.

The features of various embodiments of the present disclosure can be partially or entirely bonded to or combined with each other and can be interlocked and operated in technically various ways understood by those skilled in the art, and the embodiments can be carried out independently of or in association with each other.

For clarity of interpretation of the present specification, terms used in the present specification will be defined below.

The term "target" used in the specification may be a specific DNA or RNA. Desirably, the target may be an RNA for a specific virus, but is not limited thereto.

The term "microdroplets" used in the present disclosure is microdroplets for digital assay and may include targets (or non-target) to be amplified, a fluorescent material, or a sample for a specific reaction such as PCR.

At this time, the microdroplet may be produced by contact of a sample with oil having an immiscible property.

The term "chamber or channel" used in this specification may refer all configurations which receive microdroplets or allow the microdroplets to move therethrough. That is, the chamber or channel should not be construed as being limited to a specific structure. In the meantime, the chamber or channel includes a detection region for detecting targets in the microdroplets.

At this time, the "detection region" may refer to a region in which a plurality of microdroplets is dispersed in a plurality of columns to fill the detection region. For example, the detection region may correspond to a chamber or channel region which is detectable from an image sensor. In the meantime, when the microdroplets are present in a channel having a width corresponding to a diameter of the microdroplets, the plurality of microdroplets may be dispersed in one column.

At this time, a height of the detection region may be one time to about two times of the diameter of the plurality of microdroplets.

The term "height" used in the specification may refer to a vertical distance from an upper surface of the channel or the chamber to a lower surface.

The term "approximately" used in the specification may refer to a range of ±10% from a specific numerical value.

The term "single layer image of microdroplet" used in the specification may refer to an image for a plurality of microdroplets which includes or does not include targets or fluorescent materials as a single layer. That is, a single layer image of the microdroplets is an image for microdroplets which is aligned as the single layer on the chamber or the channel and may refer to an image captured from an upper portion of the chamber or the channel.

In the meantime, the single layer image of the microdroplets may refer to an image for the microdroplets in the chamber or the channel, and desirably, an image for a detection region of the chamber or the channel, but, it is not limited thereto. At this time, the single layer image of the microdroplets may include all an image for microdroplets stopped on the channel or chamber and an image for microdroplets which are moving as a single layer in the chamber or channel. Moreover, the single layer image of microdroplets may be a fluorescence image by microdroplets which express fluorescence by amplifying the targets, but is not limited thereto.

At this time, the image for microdroplets may include positive microdroplets and negative microdroplets.

The term "positive microdroplets" used in the specification refers to microdroplets having the target and the fluorescent material and "negative microdroplets" refer to microdroplets containing only a fluorescent material or void droplets. At this time, the positive microdroplets may express fluorescence by amplifying the targets.

The term "prediction model" used in the present disclosure may be a model trained to segment microdroplets which express fluorescence in an image with the image for microdroplets as an input.

To be more specific, the prediction model may be a model trained to segment and classify positive microdroplets and negative microdroplets or a background region in which there is no microdroplet with a fluorescence image for the microdroplets as an input.

According to the feature of the present disclosure, the prediction model may be further trained to output quantitative data (for example, copy number or a concentration) of targets based on the segmented result.

At this time, the prediction model of the present disclosure may be a model based on a ResNet deep neural network (DNN), but, it is not limited thereto. For example, the prediction model may be a SegNet network, VGG-16, a deep convolutional neural network (DCNN), a convolutional neural network (CNN), a recurrent neural network (RNN), a restricted Boltzmann machine (RBM), a deep belief network (DBN), a single shot detector (SSD) model or a U-net based prediction model.

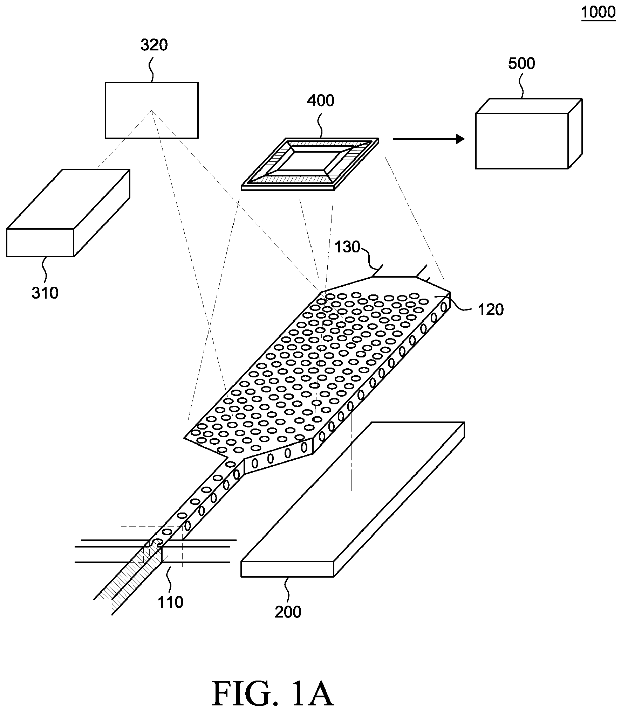

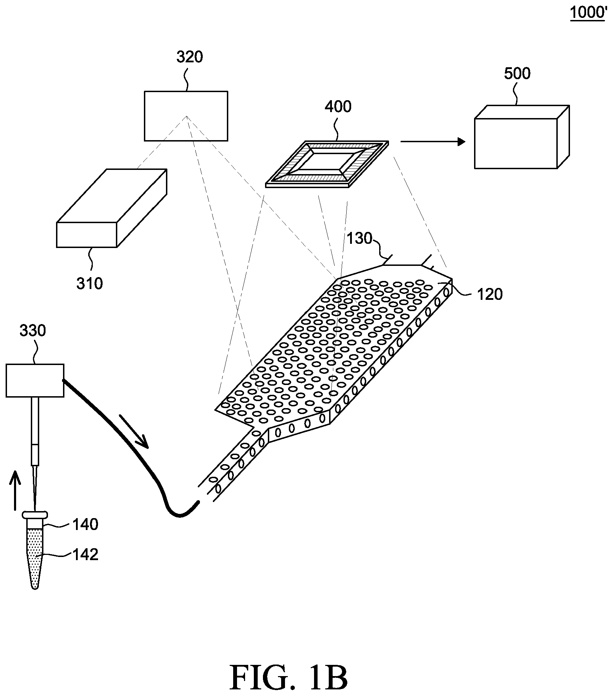

Hereinafter, a device for providing quantitative information for targets according to various exemplary embodiments of the present disclosure and a configuration thereof will be described in detail with reference to FIGS. 1A and 1B.

At this time, among digital assay methods, digital PCR will be described as an example, but is not limited thereto.

FIGS. 1A and 1B exemplarily illustrate a structure and configurations of a device for providing quantitative information for targets used for various exemplary embodiments of the present disclosure.

First, referring to FIG. 1A, a device 1000 for providing quantitative information for targets according to an exemplary embodiment of the present disclosure may be configured by a microdroplet producing unit 110 which produces microdroplets, a chamber 120 in which PCR is performed on the produced microdroplets and microdroplets on which amplification on the targets is completed is included, a valve 130 which controls the movement of the microdroplets, a light source 310, an image sensor 400 which provides an image for the microdroplets, and a processor 500 configured to communicate thereto.

At this time, the device 1000 for providing quantitative information for targets according to the exemplary embodiment of the present disclosure may further include a temperature adjusting unit 200 which controls a temperature of the chamber 120 and a reflector 320 which switches a direction of the light source.

To be more specific, the light is irradiated onto microdroplets on the chamber 120 (or the detection region), specifically, microdroplets on which the amplification is completed by the temperature adjusting unit 200, by the light source 310 and the reflector 320, and the single layer image for the plurality of microdroplets in the chamber 120 may be acquired from the image sensor 400. At this time, the height of the chamber may be one time to about two times of the diameter of the plurality of microdroplets. Therefore, the plurality of microdroplets on the chamber 120, specifically, on the detection region may be provided as a single layer.

At this time, the light source 310 may be a fluorescent lamp for expressing a color of a fluorescent material, but is not limited thereto. For example, the fluorescence may be irradiated on the chamber 120 by a fluorescence filter (not illustrated).

In the meantime, the single layer image of the microdroplets may include all an image for microdroplets stopped on the channel or chamber and an image for microdroplets which are moving as a single layer in the chamber or channel. Moreover, the single layer image of microdroplets may be a fluorescence image by microdroplets which express fluorescence by amplifying the targets, but is not limited thereto.

The single image of the microdroplets acquired from the image sensor 400 is transmitted to the processor 500 configured to communicate with the image sensor 400 and quantitative information of targets may be provided based on the image by the processor 500.

At this time, the processor 500 may perform an operation based on an artificial neural network based prediction model.

To be more specific, the processor 500 may be further configured to segment types of the microdroplets in the microdroplet image, count positive microdroplets which express the targets and negative microdroplets, and determine a concentration of targets, specifically, target genes based on the counted microdroplets, by a prediction model trained to detect a region of the microdroplets with the image for the microdroplets as an input.

According to the above-described structural feature, the quantitative analysis may be possible only with the microdroplets on which the PCR is completed, without adjusting a movement procedure of the microdroplets to a channel type detecting unit, and spacing the microdroplets. Specifically, the prediction model is applied to improve the reliability of the target testing.

In the meantime, as described above, the device 1000 for providing quantitative information for targets according to the exemplary embodiment of the present disclosure may be applied to various digital assays other than digital PCR with configurations excluding the temperature adjusting unit 200.

Moreover, the device 1000 for providing quantitative information may be configured to receive microdroplets on which reaction or analysis is completed, on the chamber 120 and generate and provide quantitative information for the targets based on the single image therefor.

For example, referring to FIG. 1B, the device 1000' for providing quantitative information may further include a tube 140 and an injector 330. To be more specific, the plurality of microdroplets 142 including target genes (targets) amplified by the PCR reaction may be received on the tube 140. At this time, the plurality of microdroplets 142 may be sucked by the injector 330 and then injected onto the chamber 120 including the detection region. At this time, a separate procedure for spacing between microdroplets such as oil injection may not be requested while injecting the plurality of microdroplets 142 into the chamber 120. Thereafter, the single image for the plurality of microdroplets 142 including amplified targets on the chamber 120 may be acquired by the image sensor 400.

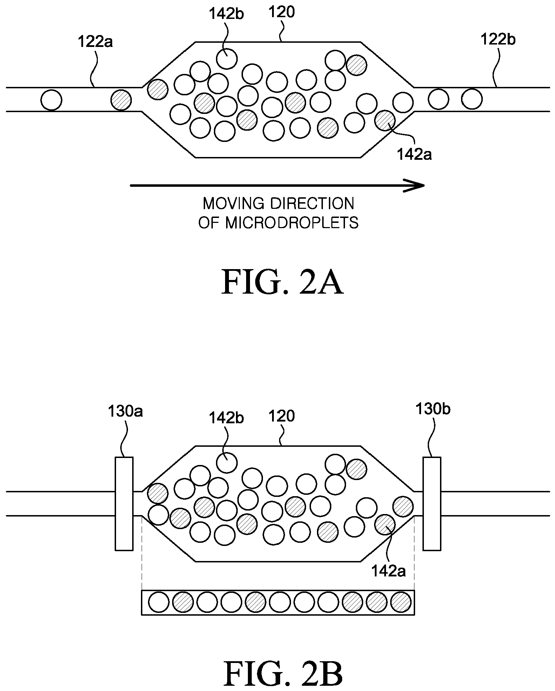

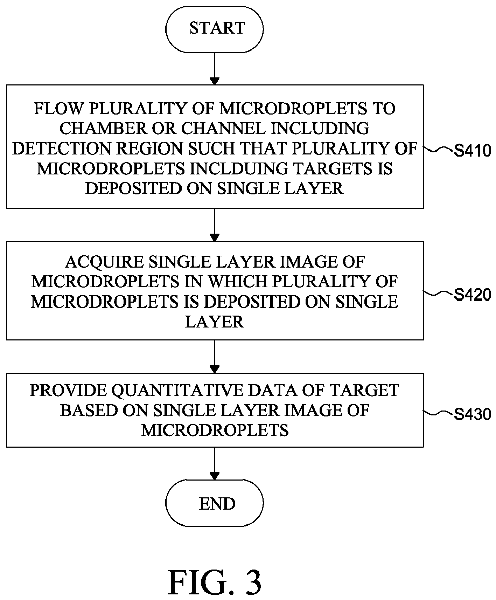

Hereinafter, a structural feature of a chamber or a channel of a device for providing quantitative information for targets used for various exemplary embodiments of the present disclosure will be described with reference to FIGS. 2A to 2C, 3A, and 3B.

FIGS. 2A to 2C exemplarily illustrate a structure and configurations of a chamber of a device for providing quantitative information for targets used for various exemplary embodiments of the present disclosure.

First, referring to FIG. 2A, the chamber 120 includes an inlet 122a through which the plurality of microdroplets 142 flows into the chamber 120 and an outlet 122b through which the plurality of microdroplets 142 is discharged to the outside of the chamber. At this time, the outlet 122b may communicate with a discarding unit (not illustrated). In the meantime, the chamber 120 may have a tapered structure in which the width is reduced toward the inlet or the outlet to smoothly flow the microdroplets. The plurality of microdroplets 142 on the chamber 120 may move in one direction by the structural feature.

In the meantime, the plurality of microdroplets 142 may be dispersed in a plurality of columns in the detection region of the chamber 120. At this time, the microdroplets may include positive microdroplets 142a which include targets (for example, target genes) and negative microdroplets 142b which do not include targets.

The above-described image sensor may sense the single layer image for the plurality of microdroplets 142 which moves in one direction in the chamber 120, specifically, in the detection region.

Referring to FIG. 2B, according to various exemplary embodiments, the inlet 122a and the outlet 122b may further include valves 130a and 130b, respectively. Therefore, the image sensor may sense the single layer image for the plurality of microdroplets 142 which is stopped in the chamber 120, specifically, in the detection region.

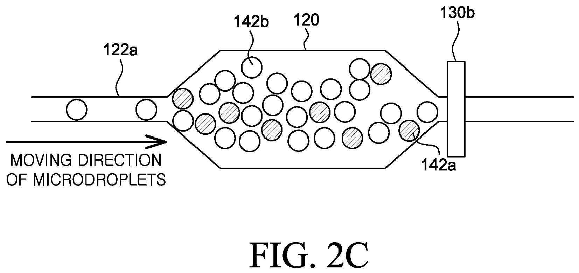

Further referring to FIG. 2C, according to various exemplary embodiments, a valve 130b may be provided only in the outlet 122b of the chamber 120. Therefore, the image sensor may sense the single layer image for the plurality of microdroplets 142 which flows through the inlet 122a.

That is, according to the structural feature having a depth which is one time to about two times of the diameter of the microdroplets, the single layer image for the plurality of microdroplets 142 which is present in various states on the chamber 120 may be acquired.

At this time, the single layer image may be an image in which the microdroplets are dispersed as a single layer on the chamber while forming a plurality of columns, but is not limited thereto.

Hereinafter, a procedure of a method for providing quantitative information for targets using a device for providing quantitative information for targets according to various exemplary embodiments of the present disclosure will be described with reference to FIGS. 3, 4A, 4B, and 5.

FIGS. 3, 4A, 4B, and 5 exemplarily illustrate procedures of providing quantitative information for targets using a device for providing quantitative information for targets according to various exemplary embodiments of the present disclosure.

First, referring to FIG. 3, according to the quantitative information providing method for targets according to the exemplary embodiment of the present disclosure, the plurality of microdroplets moves to a chamber or a channel including a detection region in step S410 and a single layer image of the microdroplets is acquired in step S420. Next, quantitative data of the targets is provided based on the single layer image of the microdroplets in step S430.

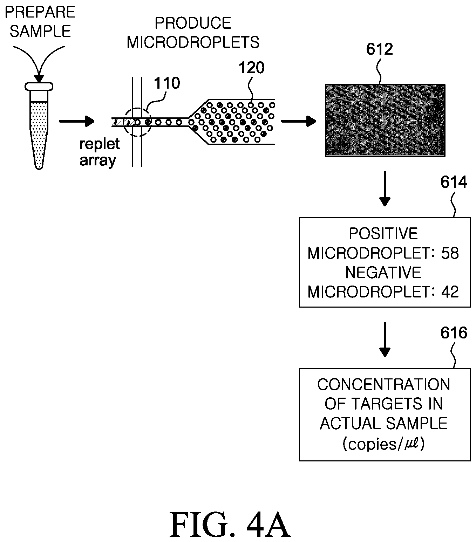

Referring to FIG. 4A together, according to various exemplary embodiments, before the step S410 of moving the plurality of microdroplets to a chamber or a channel including a detection region, in a microdroplet producing unit 110, a plurality of microdroplets encapsulated with oil by the contact of the sample including targets, fluorescent materials, a primer, and polymerase and oil is formed. As a result, the plurality of microdroplets including targets is produced.

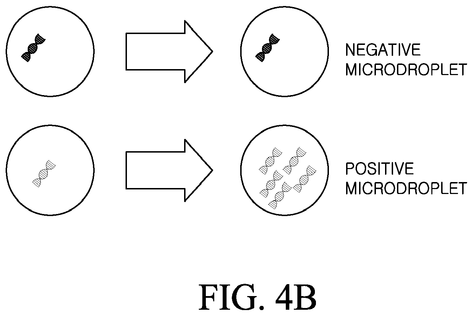

Next, in the step S410 of moving the plurality of microdroplets to the chamber or the channel including the detection region, the plurality of microdroplets moves to the chamber 120. At this time, the height of the chamber 120 may be one time to about two times of the diameter of the microdroplets. That is, the plurality of microdroplets on the chamber 120 may be present as a single layer. Next, a temperature condition cycle for amplifying the targets in the microdroplets on the chamber is provided and as a result, there may be microdroplets including an amplified targets in the chamber 120. To be more specific, referring to FIG. 4B together, as a result of controlling a temperature, the positive microdroplets including targets such as virus genes may express the fluorescence by amplifying the genes and by the fluorescent material. In contrast, in the case of the negative microdroplets which are void microdroplets, even though the PCR is performed, the fluorescence is not expressed.

Returning to FIG. 4A again, in the step S420 of acquiring the single layer image of the microdroplets, the single layer image 612 of the microdroplets which are dispersed in a plurality of columns in the chamber 120, specifically, in the detection region, are acquired by an image sensor (not illustrated) and a light source (not illustrated). At this time, the single layer image 612 of the microdroplets may be a fluorescence image. That is, the positive microdroplets having fluorescence and the negative microdroplets may be distinguished by the single layer image 612 of the microdroplets. Moreover, the single layer image 612 of the microdroplets may include an image for the microdroplets stopped on the chamber 120 or an image for microdroplets moving in the chamber 120.

Next, in the step S430 of providing quantitative data of the targets, the number of positive microdroplets and the number of negative microdroplets, that is, a counting result 614 is determined and a concentration 616 of the targets is determined. At this time, the concentration 616 of the targets may be provided as quantitative data of the targets.

According to various exemplary embodiments, the step S430 of providing quantitative data of targets may be performed by a prediction model.

To be more specific, in the step S430 of providing quantitative data of targets, regions of the positive microdroplets and the negative microdroplets for the single layer image of the microdroplets may be predicted by the prediction model.

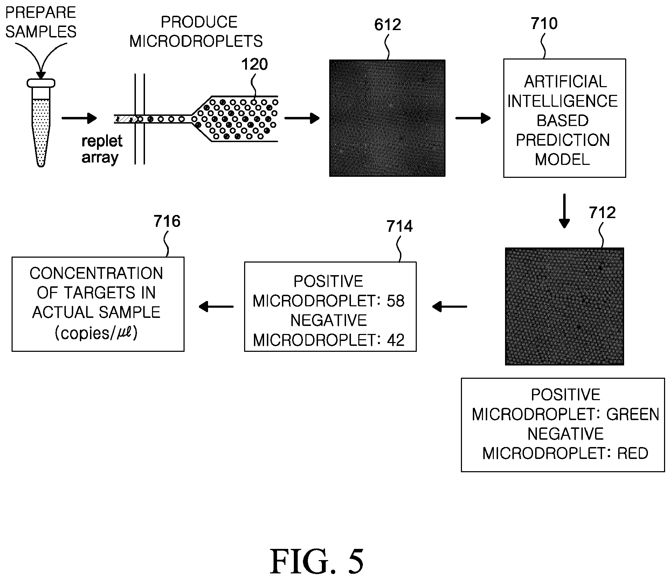

Referring to FIGS. 3 and 5 together, in the step S430 of providing quantitative data of targets, the single layer image 612 of the microdroplets acquired as the result of the step S420 of acquiring a single layer image of the microdroplets is input to an artificial neural network based prediction model 510. Next, the regions for the droplets of the positive microdroplets and the negative microdroplets and the background region may be segmented to be output by the prediction model 710. At this time, referring to the image 712 in which the microdroplet region is segmented, the negative microdroplets have red and the positive microdroplets including the target genes and the fluorescent material may have green by the expression of the fluorescent material. That is, when the single layer image 612 of the microdroplets is input, the prediction model 510 may segment the image for the microdroplets with a high accuracy.

In the meantime, the prediction model 710 may be based on a deep learning algorithm to segment an image based on ResNet, Segnet, Unet, faster rcnn, FCN, or Voxnet, but is not limited thereto.

Next, the number of positive microdroplets and negative microdroplets is determined (714) and copy number of targets may be determined based on the number of positive microdroplets and negative microdroplets. To be more specific, a concentration (copies/µl) of targets per specimen is determined based on the number of positive microdroplets and the number of negative microdroplets and the concentration of the targets may be provided as quantitative data.

In the meantime, the prediction model 710 may be a model trained to output quantitative analysis result (for example, copy number, the concentration of targets, or the number of positive microdroplets) with a single layer image of microdroplets as an input. Therefore, in the step S430 of providing quantitative data of the targets, copy number of targets, and further the concentration of the targets may be determined by the prediction model.

A precise analysis result for the targets may be provided by the quantitative information providing method according to various exemplary embodiments of the present disclosure as described above.

Specifically, the present disclosure may overcome the limitations of the digital analysis method technique of the related art in that as the intensity of the fluorescent material for each of microdroplets flowing to a detection channel is analyzed, the spacing of the microdroplets is essentially requested and it takes a long time to analyze.

That is, according to the present disclosure, the targets may be detected more easily than the reading method based on a serial counting method which requires an expensive photo multiplier tube (PMT), requests the reproduction of liquid droplets, and causes an error of quantitative analysis due to optical coherence.

Although the exemplary embodiments of the present disclosure have been described in detail with reference to the accompanying drawings, the present disclosure is not limited thereto and may be embodied in many different forms without departing from the technical concept of the present disclosure. Accordingly, the various exemplary embodiments disclosed herein are not intended to limit the technical spirit of the present disclosure but describe with the true scope and spirit being indicated by the following claims and the scope of the technical spirit of the present disclosure is not limited to the exemplary embodiments. Thus, it is to be appreciated that embodiments described above are intended to be illustrative in every sense, and not restrictive. The protective scope of the present disclosure should be construed based on the following claims, and all the technical concepts in the equivalent scope thereof should be construed as falling within the scope of the present disclosure.

## Claims

1. A quantitative information providing method for targets, comprising:
flowing a plurality of microdroplets into a chamber or a channel including a detection region such that the plurality of microdroplets including targets is present as a single layer;
acquiring a single layer image of microdroplets in which the plurality of microdroplets is present as a single layer; and
providing quantitative data of targets based on the single layer image of the microdroplets,
wherein the detection region has a height which is one time to about two times of a diameter of the plurality of microdroplets and the detection region is defined as a region in which the plurality of microdroplets is dispersed in a plurality of columns to fill the detection region.
Which is one time or more

2. The quantitative information providing method according to claim 1, wherein the chamber or the channel further includes a valve which controls the movement of the plurality of microdroplets,
wherein the acquiring of a single layer image of microdroplets further includes:
adjusting the valve to stop the plurality of microdroplets in the chamber or the channel; and
acquiring a chamber or channel image in which the plurality of stopped microdroplets is present.

3. The quantitative information providing method according to claim 1, wherein the chamber or the channel includes:
an inlet which the plurality of microdroplets flows into the chamber or the channel; and
an outlet which the plurality of microdroplets is discharged to the outside of the chamber or the channel,
wherein the flowing includes:
flowing the plurality of microdroplets from the inlet to the outlet, and
wherein the acquiring of a single layer image of microdroplets further includes:
acquiring a chamber or channel imagein which the plurality of microdroplets moves from the inlet to the outlet.

4. The quantitative information providing method according to claim 3, wherein the chamber has a tapered structure having a width which is reduced toward the inlet or the outlet.

5. The quantitative information providing method according to claim 1, further comprising:
before the flowing,
inducing the contact of a sample including the targets and the fluorescent material, and oil having an immiscible property with the sample to produce the plurality of microdroplets, and
after the flowing,
controlling a temperature to perform polymerase chain reaction (PCR) of the targets in the plurality of microdroplets in the chamber or the channel,
wherein the acquiring of a single layer image of microdroplets further includes:
acquiring a single layer image of the plurality of microdroplets including the amplified targets.

6. The quantitative information providing method according to claim 1, further comprising:
before the flowing,
inducing the contact of a sample including the targets and the fluorescent material and oil having an immiscible property with the sample to produce the plurality of microdroplets; and
controlling a temperature to perform PCR of the targets in the plurality of microdroplets,
wherein the flowing includes:
flowing the plurality of microdroplets including the amplified targets to the chamber or the channel without spacing the microdroplets.

7. The quantitative information providing method according to claim 1, wherein the providing of quantitative data of the targets includes:
predicting a region of a positive microdroplet and a region of a negative microdroplet based on the single layer image of the microdroplets using an artificial neural network based prediction model configured to segment the regions of the positive microdroplets and the negative microdroplets with the single layer image of the microdroplets as an input;
determining a number for the plurality of microdroplets based on the region of the positive microdroplets and the region of the negative microdroplets; and
providing quantitative data of targets based on the number of the plurality of microdroplets.

8. The quantitative information providing method according to claim 7, wherein the positive microdroplets are defined as microdroplets including the targets and fluorescent materials,
the negative microdroplets are defined as microdroplets including only the fluorescent materials or void droplets, and
wherein the providing of quantitative data of the targets further includes:
determining a concentration of the targets based on the number of positive microdroplets and negative microdroplets.

9. The quantitative information providing method according to claim 7, wherein the prediction model is further configured to quantitatively analyze the targets based on the region of the positive microdroplets and the region of the negative microdroplets, and
wherein the providing of quantitative data of the targets further includes:
determining quantitative data of targets based on the number of the plurality of microdroplets using the prediction model.

10. A device for providing quantitative information for targets, comprising:
a chamber or a channel which receives a plurality of microdroplets including targets and includes a detection region in which the plurality of microdroplets is present as a single layer;
a light source which irradiates light in the detection region of the chamber or the channel;
an image sensor configured to provide a single layer image of the microdroplets in which the plurality of microdroplets is present as the single layer in the detection region; and
a processor which is operably connected to the image sensor,
wherein the processor is configured to provide quantitative data of targets based on the single layer image of the microdroplets, and the detection region has a height which is one time to about two times of a diameter of the plurality of microdroplets and the detection region is defined as a region in which the plurality of microdroplets is dispersed in a plurality of columns to fill the detection region.

11. The device for providing quantitative information according to claim 10, wherein the chamber or the channel further includes a valve which controls the movement of the plurality of microdroplets,
wherein the processor is configured to adjust the valve to stop the plurality of microdroplets in the chamber or the channel; and
wherein the image sensor is configured to acquire a chamber or channel image in which the plurality of stopped microdroplets is present.

12. The device for providing quantitative information according to claim 10, wherein the chamber or the channel includes:
an inlet which the plurality of microdroplets flows into the chamber or the channel; and
an outlet which the plurality of microdroplets is discharged to the outside of the chamber or the channel,
wherein the processor is configured to flow the plurality of microdroplets from the inlet to the outlet, and
wherein the image sensor is configured to acquire a chamber or channel image in which the plurality of microdroplets moves from the inlet to the outlet.

13. The device for providing quantitative information according to claim 12, wherein the chamber has a tapered structure having a width which is reduced toward the inlet or the outlet.

14. The device for providing quantitative information according to claim 10, further comprising:
a microdroplet producing unit configured to induce the contact of a sample including the targets and the fluorescent material and oil having an immiscible property with the sample to produce the plurality of microdroplets; and
a temperature adjusting unit configured to control a temperature to perform polymerase chain reaction (PCR) of the targets in the plurality of microdroplets in the chamber or the channel,
wherein the image sensor is further configured to acquire a single layer image of the microdroplets including an amplified target.

15. The device for providing quantitative information according to claim 10, further comprising:
a microdroplet producing unit configured to induce the contact of a sample including the targets and the fluorescent material and oil having an immiscible property with the sample to produce the plurality of microdroplets; and
a temperature adjusting unit configured to control a temperature to perform PCR of the targets in the plurality of microdroplets,
wherein in the chamber or the channel, the plurality of microdroplets including the amplified targets moves or is received without spacing the microdroplets.
